# FASCICULE DE BREVET EUROPEEN

(11) **EP 2 178 504 B1**
(45) Date de publication et mention de la délivrance du brevet: **05.01.2011**
(21) Numéro de dépôt: 08826597.0
(22) Date de dépôt: 17.07.2008
(51) Int. Cl.: A61K 9/08, A61K 31/5575, A61K 47/14, A61P 27/06

(54) **SOLUTION OPHTALMIQUE À BASE DE PROSTAGLANDINES SANS CONSERVATEUR**
KONSERVIERUNGSMITTELFREIE AUGENLÖSUNG AUF PROSTAGLANDIN-BASIS
PRESERVATIVE-FREE PROSTAGLANDIN-BASED OPHTHALMIC SOLUTION

(30) Priorité: 20.07.2007 FR 0705272
(43) Date de publication de la demande: 28.04.2010
(73) Titulaire: Laboratoires THEA, 63100 Clermont-Ferrand (FR)
(72) Inventeur: PILOTAZ, Frédéric, F-38410 Vaulnaveys Le Haut (FR); MERCIER, Fabrice, F-63000 Clermont-Ferrand (FR); CHIBRET, Henri, F-63350 Joze (FR)
(74) Mandataire: Denjean, Eric
(86) Numéro de dépôt international: PCT/FR2008/051347
(87) Numéro de publication internationale: WO 2009/013435

(56) Documents cités:
- EP-A- 0 850 926
- EP-A- 1 666 043
- WO-A-91/12008
- US-A- 4 431 833
- US-A1- 2004 082 660

## Description

L'invention a pour objet un collyre ou solution ophtalmique comprenant en tant que principe actif, au moins une prostaglandine, ladite solution étant exempte d'agent conservateur de type anti-microbien, en particulier du type ammonium quaternaire (par exemple chlorure de benzalkonium (BAK)).

Dans la suite de la description, par « agent conservateur de type anti-microbien ou anti-microbien», on désigne un agent conservateur présentant des propriétés anti-microbiennes, c'est-à-dire un composé capable de garantir la protection de la solution ophtalmique vis-à-vis d'une éventuelle contamination microbienne. Un tel agent au sens de l'invention est à distinguer des agents conservateurs agissant sur la conservation chimique de la solution tels que par exemple des agents antioxydants comme l'EDTA.

Les prostaglandines sont des principes actifs bien connus qui sont administrés à l'homme ou à l'animal par voie topique sous forme de collyre pour le traitement du glaucome. La posologie usuelle de ces formules est de 1 goutte par jour dans les deux yeux, sachant que les prostaglandines peuvent être également utilisées en association avec un deuxième agent anti-glaucomateux tel que par exemple, béta-bloquant, inhibiteur de l'anhydrase carbonique ou encore agoniste alpha-adrénergique.

Les prostaglandines présentent un premier inconvénient qui est celui de ne pas être solubles dans l'eau, de sorte que leur mise en solution au sein du collyre passe nécessairement par une étape préalable de solubilisation. Par ailleurs, une autre contrainte pour le formulateur est de proposer une solution ophtalmique qui soit chimiquement stable dans le temps, en pratique sur une période comprise entre 18 et 24 mois. Une autre caractéristique requise de la solution ophtalmique est d'être stable vis-à-vis du conditionnement dans lequel elle est stockée, en particulier vis-à-vis des conditionnements en matériau plastique du type polyéthylène de base densité (PEBD).

Actuellement, la plupart des solutions ophtalmiques à base de prostaglandines sur le marché renferment un agent conservateur qui, outre ses propriétés anti-microbiennes, assure également la solubilisation du principe actif et en partie sa stabilisation. Il s'agit par exemple du produit commercialisé sous la marque Xalatan® par la société PFIZER associant Latanoprost et BAK à hauteur de 0,02 % en poids. Il est à noter que malgré la présence de BAK, ce collyre n'est pas stable à température ambiante et doit être stocké au froid, à une température de l'ordre de 5° C. La société ALLERGAN commercialise par ailleurs un collyre sous la marque Lumigan® associant Bimatoprost et BAK à hauteur de 0,005 % en poids.

Toutefois, de nombreuses publications déconseillent l'usage de conservateur de type anti-microbien, en particulier le BAK, en ophtalmologie dans le cadre de traitements au long cours comme c'est le cas notamment du glaucome pour des problèmes de tolérance (voir en ce sens The New Class of Ophthalmic Agents: Here's how to choose the right prostaglandin for the each patient " by J. JAMES THIMONS, O.D., F.A.A.O. - Optometric management, issue May 2002. Il est donc dorénavant établi que les agents conservateurs de type anti-microbien s'avèrent toxiques lors d'un usage au long cours, de sorte que l'on tend aujourd'hui à limiter leur usage en réduisant au maximum leur concentration dans les collyres, ou mieux, à les éliminer des formules.

Cette problématique a été prise en considération dans le document WO 97/29752, qui divulgue l'utilisation en lieu et place d'une partie du BAK, d'un agent non ionique du type Cremophor®. Dans la formule proposée, la concentration en BAK est limitée à 0,01 % en poids, la concentration de Cremophor® EL étant de 0,05 % en poids. Sur le marché, on trouve un produit dénommé Travatan^{®} associant Travoprost, BAK et Cremophor®, commercialisé par la société ALCON titulaire de la demande de brevet WO/9729752.

Le polysorbate 80 a également été proposé dans des solutions ophtalmiques pour se substituer partiellement au BAK, comme c'est par exemple le cas du produit commercialisé sous la marque Rescula^{®} par la société NOVARTIS associant unoprostone avec un mélange BAK et polysorbate 80 représentant 0,015 % en poids de la solution.

Les documents EP 1 321 144 et EP1 666 043 décrivent quant à eux la capacité du polysorbate 80 à plus ou moins ralentir l'adsorption d'une prostaglandine spécifique, en l'espèce le tafluprost successivement sur le polyéthylène (PE), le polyéthylène basse densité (PEBD), le polypropylène (PP), le polyéthylène terephtalate (PET), un mélange PET/polyarylate constitutif du conditionnement. Aucune indication n'est donnée concernant l'éventuelle capacité du polysorbate 80 à stabiliser la solution dans le temps sur une période de 18 à 24 mois. Ces documents ne contiennent aucun exemple de solution ophtalmique prête à l'emploi et précise simplement qu'un certain nombre d'additifs peuvent être ajoutés à la combinaison prostaglandine et polysorbate 80, tels que par exemple des agents conservateurs du type BAK. Rien n'indique dans ce document que le BAK, ou les conservateurs en général seraient expressément exclus de la composition finale.

Le document US2004/0082660 décrit une solution ophtalmique dépourvue de BAK et contenant un mélange de latanoprost et de polysorbate 80 (table 1, test 2). Les résultats mentionnés caractérisent l'homogénéité de la solution obtenue 30 minutes après 7 heures d'agitation, c'est-à-dire la capacité du polysorbate 80 à solubiliser le latanoprost en 7 heures. Aucune indication n'est donnée concernant la stabilité chimique de la solution dans le temps.

Le document EP 0850 926 décrit des solutions ophtalmiques associant prostaglandine, polysorbate 80 et glycérine. Aucune indication n'est donnée concernant la solubilité de la prostaglandine, ainsi que la stabilité chimique de la solution dans le temps et vis-à-vis du conditionnement.

Le problème que se propose donc de résoudre l'invention est de développer une formule à base de prostaglandine qui soit dépourvue d'agent conservateur anti-microbien et qui soit stable dans le temps en solution à température ambiante (dans un délai de 18 à 24 mois), de même que vis-à-vis du conditionnement en matière plastique dans lequel elle est usuellement stockée, en particulier les conditionnements en PEBD.

Un autre objectif est de proposer une formulation suffisamment fluide pour être stérilisée par filtration et conditionnée par technique de conditionnement aseptique de type « Blow-Fill-Seal ».

Le Demandeur à constaté que le fait de substituer le polysorbate 80 par le solutol HS 15 permettait de résoudre le problème de stabilité des prostaglandines dans le temps et vis-à-vis du conditionnement primaire, en particulier les conditionnements en PEBD.

Le solutol HS15 est un produit dont la dénomination CAS est le polyoxyl-15-hydroxystéarate et dont le numéro CAS est le 70142-34-6. Ce produit commercialisé sous la dénomination Solutol^{®} HS 15 dispose également d'une monographie inscrite à la pharmacopée européenne et identifiée sous la dénomination Macrogol 15 hydroxystéarate.

Il est à noter que le Solutol^{®} HS 15 est décrit dans un document WO 91/12008 au sein d'une composition ophtalmique en combinaison avec un antibiotique appartenant à la famille des macrolides et en particulier la primycine.

Le Demandeur a constaté que de manière tout à fait surprenante, l'utilisation de Solutol^{®} HS 15 comme agent solubilisant de prostaglandine dans une solution ophtalmique permet en outre de conférer à la solution obtenue une stabilité à température ambiante pendant au moins 18 mois, mais également vis-à-vis des conditionnements, en particulier les conditionnements plastiques du type PEBD de qualité pharmacopée européenne (EP) qui sont des conditionnements pour produits stériles, fabriqués à partir de polyethylène sans additifs.

L'invention a donc pour objet une solution ophtalmique prête à l'emploi dépourvue d'agent conservateur anti-microbien comprenant en tant que principe actif au moins une prostaglandine, ainsi qu'en tant qu'agent solubilisant, un tensio-actif caractérisé en ce que l'agent solubilisant est le polyoxyl-15-hydroxystearate.

Selon une première caractéristique, la concentration de l'agent solubilisant (Solutol^{®} HS 15) dans la solution est comprise entre 1 et 20 g/l, avantageusement entre 2 et 10 g/l.

En dessous de la concentration de 0.1% (m/v), l'effet solubilisant est incomplet. Au dessus de 2% (m/v), on n'observe pas d'amélioration notoire de la solubilisation.

Selon une autre caractéristique, la concentration en prostaglandine dans la solution est comprise entre 0.02 et 1.5 g/l.

En pratique, les prostaglandines sont dans la solution au nombre d'au moins une et sont choisies dans le groupe comprenant 17-phenyl-13,14 dihydro trinor prostaglandin F₂ₐ isopropyl ester (latanoprost), 20-ethyl prostaglandin F_{2α}, (+)- fluprostenol isopropyl ester (travoprost), 17-phenyl trinor prostaglandin F_{2α} amide, 17-phenyl-13,14 dihydro trinor prostaglandin F_{2α} ethyl amide (bimatoprost), tafluprost prostaglandin F_{2α} ethanolamide, bimatoprost (freeacid)-d₄, bimatoprost-d₄, latanoprost ethyl amide, 13,14 dihydro-15-keto-20-ethyl prostaglandin F_{2α} (unoprostone), 13,14 dihydro-15-keto-20-ethyl prostaglandin F_{2α} isopropyl ester (unoprostone isopropyl ester).

Comme déjà dit, la ou les prostaglandines peuvent être associées à un second principe actif en particulier avec des agents anti-glaucomateux d'autres classes pouvant ainsi agir de manière synergique. Il peut s'agir par exemple des béta-bloquants choisis dans le groupe comprenant le maléate de timolol et le chlorure de carteolol, des inhibiteurs de l'anhydrase carbonique tels que ceux par exemple choisis dans le groupe comprenant le chlorure de dorzolamide ou encore des agonistes alpha-adrénergiques tels que par exemple le tartrate de brimonidine. Des exemples d'associations de prostaglandines et de béta-bloquants sont par exemple :
Xalacom^{®} - Pfizer : Latanoprost 0,005% + Timolol 0,5%,
Ganfort^{®} - Allergan : Bimatoprost 0,03% + Timolol 0,5%
Duatrav^{®} - Alcon : Travoprost 0,004% + Timolol 0,5%

En pratique, l'agent anti-glaucomateux représente entre 0,1 et 0,5% en poids de la solution.

Bien entendu, la composition de l'invention peut contenir des additifs usuels à l'exclusion des agents conservateurs anti-microbiens . Il peut s'agir par exemple d'agents isotonisants de type non ioniques tels que des polyols (Ex : glycérol, mannitol etsorbitol).

La formulation de l'invention peut être présentée dans des flacons à usage unique ou dans un flacon multi-doses du type par exemple Abak^{®} ou Comod^{®} ou équivalent, de tels flacons permettant de délivrer des collyres sans conservateurs pendant plusieurs jours.

Par ailleurs et comme déjà dit, le Demandeur a constaté que la solution ophtalmique de l'invention était stable vis-à-vis des conditionnements plastiques du type PEBD de qualité EP «Polyethylène sans additifs destinés au conditionnement de produit stérile » correspondant à la définition de la pharmacopée européenne.

L'invention a donc pour objet un flacon à usage unique ou multidose comprenant la solution ophtalmique précédemment décrite réalisé en PEBD de qualité EP ne contenant pas d'additifs.

La composition ophtalmique se présentant sous la forme d'une solution, celle-ci peut être stérilisée par filtration à 0,2 µm sur une membrane appropriée comme le PES ou le PVDF. En outre, cette propriété, associée à la stabilité du produit lorsqu'il est conditionné dans un récipient en PEBD, rend sa fabrication compatible avec la technologie BFS.

Bien entendu, l'invention concerne également l'utilisation de la solution ophtalmique telle que précédemment décrite pour la fabrication d'un médicament destiné au traitement du glaucome chez l'homme ou l'animal, en particulier pour sa capacité à réduire la pression intraoculaire et/ou à assurer la neuroprotection des tissus rétiniens.

En pratique, le collyre est administré à raison d'une goutte par jour dans chaque oeil.

L'invention a également pour objet une méthode de traitement thérapeutique du glaucome chez l'homme ou l'animal consistant à instiller la solution ophtalmique précédemment décrite à raison d'une goutte par jour dans chaque oeil.

L'invention et les avantages qui en découlent ressortiront mieux des exemples de réalisation suivants à l'appui des figures annexées.
La figure 1 compare l'effet stabilisant du Cemophor^{®} EL et du Solutol^{®} HS 15 à concentration identique (0.5%) vis-à-vis du travoprost.
La figure 2 compare l'effet stabilisant du Solutol^{®} HS 15 (0,5 %) et du polysorbate 20 (2 %) vis-à-vis du latanoprost.
La figure 3 compare l'effet stabilisant du Solutol^{®} HS 15 (0,5 %) et du polysorbate 80 (2 % et 0.5%) vis-à-vis du latanoprost.
La figure 4 compare l'effet stabilisant du Solutol^{®} HS 15 (0,5 %) vis-à-vis du BAK (formulation Xalatan^{®}).

### Exemple 1 : Comparaison de l'effet stabilisant du Solutol^{®} HS 15 et du Cremophor^{®} EL vis-à-vis du travoprost

### 1 - formule à base de Solutol^{®} HS 15

| Produits | Fonctions | Formule centésimale |
|---|---|---|
| travoprost | Principe actif | 0,004 g |
| PEG-15 | Agent solubilisant et | 0,5 g |
| hydroxystearate* | stabilisant | |
| | Agent tampon | 0,317g |
| Na₂HPO₄, 12H₂O | Agent tampon | 0,067 g |
| NaH₂PO₄, 2H₂O | Agent isotonisant | 4,5 g |
| Sorbitol | Agent antioxidant | 0,05 g |
| EDTA | Véhicule | qsp 100 g |
| Eau purifiée | | |

| | | |
|---|---|---|
| *Solutol^{®} HS 15 | | |

### 2 - formule à base de Cremophor® EL

| Produits | Fonctions | Formule centésimale |
|---|---|---|
| travoprost | Principe actif | 0,004 g |
| PEG-35 Castor oil* | Agent solubilisant | 0,5 g |
| Na₂HPO₄, 12H₂O | Agent tampon | 0,317g |
| NaH₂PO₄, 2H₂O | Agent tampon | 0,067 g |
| Sorbitol | Agent isotonisant | 4.5 g |
| EDTA | Agent antioxidant | 0,05 g |
| Eau purifiée | Véhicule | qsp 100 g |

| | | |
|---|---|---|
| * nom commercial : Cremophor^{®} EL | | |

### 3 - Mode de préparation des formules

- Introduire de l'eau PPI dans la cuve inox du mélangeur.
- Effectuer un barbotage à l'azote afin d'éliminer au maximum l'oxygène dissous dans l'eau. Maintenir l'inertage d'azote tout au long de la fabrication.
- Ajouter et dissoudre, en maintenant l'agitation, les excipients : agent isotonisant, agent tampon, agent antioxydant et agent solubilisant.
- Chaque ingrédient doit être complètement dissous avant l'addition du produit suivant.
- Ajouter et dissoudre, en maintenant l'agitation, le principe actif.
- Lorsque l'homogénéisation est totale, l'ajustement en poids est effectué avec de l'eau PPI.
- Effectuer la filtration stérilisante à l'aide d'un filtre PES ou PVDF 0,2 µm sous flux d'azote.

### 4 - Conditionnement

Les solutions stériles ainsi préparées sont conditionnées en flacons plastiques de type PEBD.

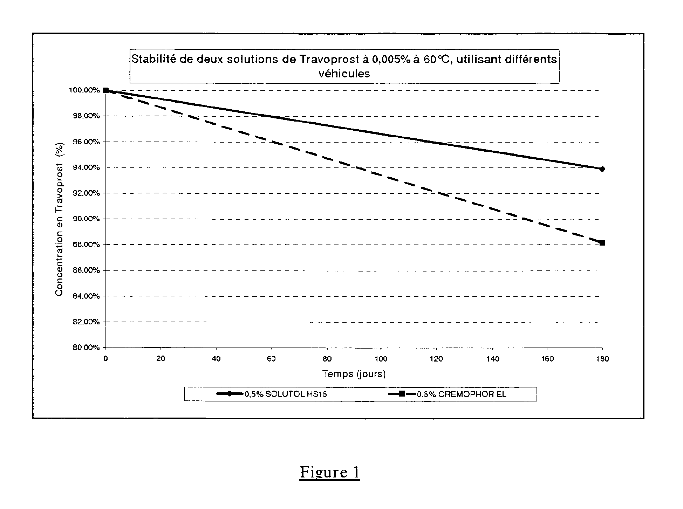

Comme le montre la figure 1, à concentration égale, l'effet stabilisant du Solutol^{®} HS 15 vis-à-vis du travoprost à une concentration de 0,004 % est meilleur que celui du Cremophor^{®}. En particulier, après 180 jours de conservation dans une enceinte climatique à 60°C, la concentration de travoprost est de 96 % dans la formule au Solutol^{®} HS 15 et de seulement 88 % dans la formule au Cremophor^{®} EL.

### Exemple 2 : Stabilité du Solutol^{®} HS 15 et du polysorbate 20 vis-à-vis du latanoprost

Les deux formules suivantes sont comparées :

### 1 - formule à base de Solutol^{®} HS 15

| Produits | Fonctions | Formule centésimale |
|---|---|---|
| latanoprost* | Principe actif | 0,005 g |
| PEG-15 hydroxystearate* | Agent solubilisant et stabilisant | 0,5 g |
| Na₂HPO₄, 12H₂O | Agent tampon | 0,317g |
| NaH₂PO₄, 2H₂O | Agent tampon | 0,067 g |
| Sorbitol | Agent isotonisant | 4,5 g |
| EDTA | Agent antioxidant | 0,05 g |
| Eau purifiée | Véhicule | qsp 100 g |

| | | |
|---|---|---|
| * latanoprost sous la forme de isopropyl ester *Solutol^{®} HS 15 | | |

### 2 - formule à base de volysorbate 20

| Produits | Fonctions | Formule centésimale |
|---|---|---|
| latanoprost (isopropyl ester) | Principe actif | 0,005 g |
| Polysorbate 20* | Agent solubilisant | 2,0 g |
| Na₂HPO₄, 12H₂O | Agent tampon | 0,317 g |
| NaH₂PO₄, 2H₂O | Agent tampon | 0,067 g |
| Sorbitol | Agent isotonisant | 3,5 g |
| EDTA | Agent antioxidant | 0,05 g |
| Eau purifiée | Véhicule | qsp 100 g |

| | | |
|---|---|---|
| * Nom commercial MONTANOX^{®} 20PHA | | |

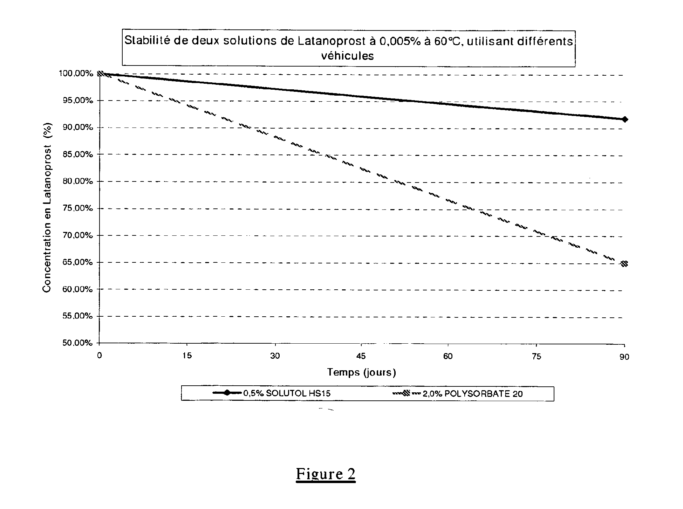

Comme le montre la figure 2, à concentration inférieure (0,5 % contre 2 %), le Solutol^{®} HS 15 a un effet stabilisant vis-à-vis du latanoprost supérieur à celui du polysorbate 20. En particulier, après 90 jours de conservation dans une enceinte climatique à 60°C, la concentration résiduelle en latanoprost est de plus de 90 % dans le cas de la formule au Solutol^{®} HS 15, alors qu'elle est de seulement 65 % dans le cas de la formule au polysorbate 20.

### Exemple 3 : Stabilité du Solutol^{®} HS 15 et du polvsorbate 80 vis-à-vis du latanoprost

### 1 - formule à base de Solutol^{®} HS 15

| **Produits** | **Fonctions** | **Formule centésimale** |
|---|---|---|
| latanoprost* | Principe actif | 0,005 g |
| PEG-15 hydroxystearate* | Agent solubilisant et stabilisant | 0,5 g |
| Na₂HPO₄, 12H₂O | Agent tampon | 0,317 g |
| NaH₂PO₄, 2H₂O | Agent tampon | 0,067 g |
| Sorbitol | Agent isotonisant | 4,5 g |
| EDTA | Agent antioxidant | 0,05 g |
| Eau purifiée | Véhicule | qsp 100 g |

| | | |
|---|---|---|
| * latanoprost sous la forme de isopropyl ester *Solutol^{®} HS 15 | | |

### 2 - formule à base de polysorbate 80 à 2%

| **Produits** | **Fonctions** | **Formule centésimale** |
|---|---|---|
| latanoprost (isopropyl ester) | Principe actif | 0,005 g |
| Polysorbate 80* | Agent solubilisant | 2,0 g |
| Na₂HPO₄, 12H₂O | Agent tampon | 0,317 g |
| NaH₂PO₄, 2H₂O | Agent tampon | 0,067 g |
| Sorbitol | Agent isotonisant | 3,5 g |
| EDTA | Agent antioxidant | 0,05 g |
| Eau purifiée | Véhicule | qsp 100 g |

| | | |
|---|---|---|
| * Nom commercial MONTANOX^{®} 80PHA | | |

### 3 - formule à base de polysorbate 80 à 0,5%

| Produits | Fonctions | Formule centésimale |
|---|---|---|
| latanoprost (isopropyl ester) | Principe actif | 0,005 g |
| Polysorbate 80* | Agent solubilisant | 0,5 g |
| Na₂HPO₄,12H₂O | Agent tampon | 0,317 g |
| NaH₂PO₄, 2H₂O | Agent tampon | 0,067 g |
| Sorbitol | Agent isotonisant | 4,5 g |
| EDTA | Agent antioxidant | 0,05 g |
| Eau purifiée | Véhicule | qsp 100 g |

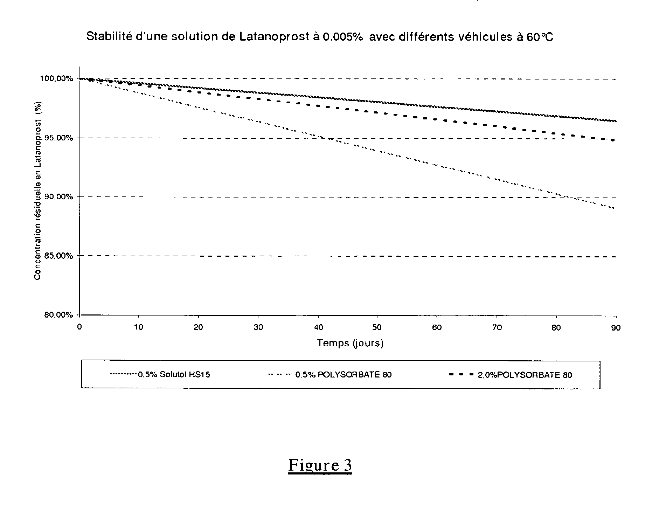

Comme le montre la figure 3, à concentration inférieure (0,5 % contre 2 %), le Solutol^{®} HS 15 a un effet stabilisant vis-à-vis du latanoprost supérieur à celui du polysorbate 80. Après 90 jours de conservation dans une enceinte climatique à 60°C, la concentration résiduelle en latanoprost est de plus de 90 % dans le cas de la formule au Solutol^{®} HS 15, c'est-à-dire au-delà de la limite requise pour considérer le produit conforme au critère de stabilité.

Pour une concentration en polysorbate 80 de 2%, la concentration résiduelle en latanoprost est également de plus de 90 % mais reste supérieure à celle obtenue en présence de solutol®HS 15. En outre, ce résultat est obtenu avec une concentration supérieure en polysorbate par rapport au solutol® HS15. La formule à 2% de polysorbate 80 présente donc plus de risques en terme d'effet secondaire, que la formule au solutol® HS15.

Pour une concentration en polysorbate 80 de 0.5%, la concentration résiduelle en latanoprost est inférieure à 90 %, ce qui rend le produit non-conforme au critère de stabilité.

### Exemple 4 : Stabilité à température ambiante (25°C /40 % HR) de la formule à base de Solutol^{®} HS 15 comparativement à une formulation avec conservateur (Xalatan®) conditionnées en flacon en PEBD de qualité EP.

Dans cette étude, on compare deux formules, respectivement :

### - formule à base de Solutol^{®} HS 15

| Produits | Fonctions | Formule centésimale |
|---|---|---|
| latanoprost* | Principe actif | 0,005 g |
| PEG-15 hydroxystearate* | Agent solubilisant et stabilisant | 0,5 g |
| Na₂HPO₄, 12H₂O | Agent tampon | 0,317g |
| NaH₂PO₄, 2H₂O | Agent tampon | 0,067 g |
| Sorbitol | Agent isotonisant | 4,5 g |
| EDTA | Agent antioxidant | 0,05 g |
| Eau purifiée | Véhicule | qsp 100 g |

| | | |
|---|---|---|
| * latanoprost sous la forme de isopropyl ester * Solutol^{®} HS 15 | | |

### - produit commercial Xalatan^{®} contenant 0,005% de latanoprost sous la forme de isopropyl ester et 0,02% de conservateur (BAK)

| | Formule Solutol (sans conservateur) *(= formule A)* | Formule Xalatan® (avec conservateur / BAK) |
|---|---|---|
| Temps initial | 100,0 % | 100,0 % |
| Temps 6 mois | 100,3% | 93,7% |

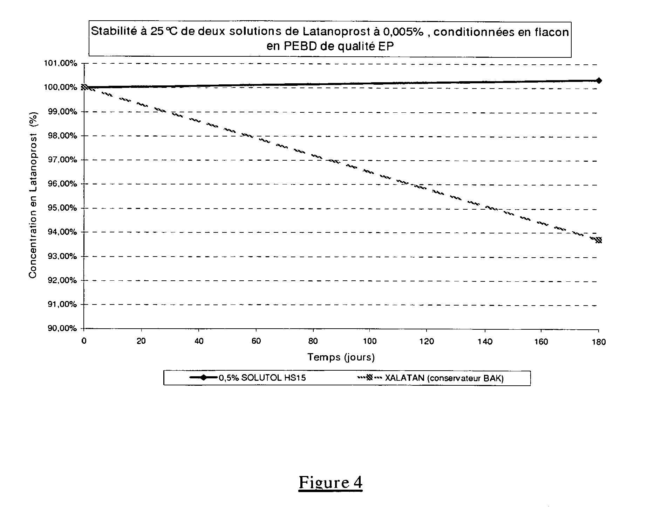

Comme le montre le tableau ci-dessus et la figure 4, la formulation avec conservateur n'est pas stable à 25°C dans un conditionnement en PEBD de qualité EP. En revanche, la solution ophtalmique de l'invention peut être commercialisée dans des conditionnements réalisés simplement en PEBD de qualité EP sans que la stabilité de ladite solution soit affectée.

### Exemple 5 : Collyre d'unoprostone à 0.1 %

| Produits | Fonctions | Formule centésimale |
|---|---|---|
| unoprostone* | Principe actif | 0,1 g |
| PEG-15-hydroxystearate* | Agent solubilisant et stabilisant | 2,0 g |
| Na₂HPO₄, 12H₂O | Agent tampon | 0,317g |
| NaH₂PO₄, 2H₂O | Agent tampon | 0,067 g |
| Sorbitol | Agent isotonisant | 3,5 |
| EDTA | Agent antioxydant | 0,05 g |
| Eau purifiée | Véhicule | qsp 100 g |

| | | |
|---|---|---|
| * Unoprostone sous la forme de isopropyl * Solutol^{®} HS 15 | | |

### Exemple 6 : Collyre latanoprost + timolol

| Produits | Fonctions | Formule centésimale |
|---|---|---|
| latanoprost (isopropyl ester) | Principe actif No 1 | 0,005 g |
| Timolol maléate | Principe actif No 2 | 0,685 g |
| PEG-15-hydroxystearate* | Agent solubilisant et stabilisant | 0,5 g |
| Na₂HPO₄, 12H₂O | Agent tampon | 0,317g |
| NaH₂PO₄, 2H₂O | Agent tampon | 0,067 g |
| Sorbitol | Agent isotonisant | 4,0 |
| EDTA | Agent antioxydant | 0,05 g |
| Eau purifiée | Véhicule | qsp 100 g |

| | | |
|---|---|---|
| * Solutol^{®} HS 15 | | |

### Exemple 7 : Collyre latanoprost + cartéolol

| Produits | Fonctions | Formule centésimale |
|---|---|---|
| latanoprost (isopropyl ester) | Principe actif | 0,005 g |
| Cartéolol (chlorydrate) | Principe actif | 2,0 g |
| PEG-15-hydrostearate* | Agent solubilisant et stabilisant | 0,5 g |
| Na₂HPO₄, 12H₂O | Agent tampon | 0,317g |
| NaH₂PO₄, 2H₂O | Agent tampon | 0,067 g |
| Sorbitol | Agent isotonisant | 3,0 |
| EDTA | Agent antioxydant | 0,05 g |
| Eau purifiée | Véhicule | qsp 100 g |

| | | |
|---|---|---|
| * Solutol^{®} HS 15 | | |

### Exemple 8 : Collyre latanoprost + dorzolamide

| Produits | Fonctions | Formule centésimale |
|---|---|---|
| Latanoprost (isopropyl ester) | Principe actif No 1 | 0,005 g |
| Dorzolamide (HCl) | Principe actif No 2 | 2,0 g |
| PEG-15-hydrostearate* | Agent solubilisant et stabilisant | 0,5 g |
| Na₂HPO₄, 12H₂O | Agent tampon | 0,317 g |
| NaH₂PO₄, 2H₂O | Agent tampon | 0,067 g |
| Sorbitol | Agent isotonisant | 3,0 |
| EDTA | Agent antioxydant | 0,05 g |
| Eau purifiée | Véhicule | qsp 100 g |

| | | |
|---|---|---|
| * Solutol^{®} HS 15 | | |

### Exemple 9 : Test d'irritation oculaire comparatif de différents collyres de Latanoprost chez l'animal

*Objectif de l'étude :* Afin d'évaluer la compatibilité de deux formules ophtalmiques sans conservateur de prostaglandines avec la surface oculaire, un test d'irritation est réalisé chez le lapin : test de Draize.

*Méthodologie :* Le test est réalisé de manière comparative contre un produit de référence, dont la compatibilité avec la surface oculaire est considérée comme acceptable : Xalatan^{®} - collyre commercial. Un témoin négatif est également suivi (solution de NaCl à 0,9%).

*Protocole :* Différents critères d'évaluation sont enregistrés après plusieurs instillations des produits à tester. 12 lapins, mâles albinos (blancs de Nouvelle Zélande)) sont répartis en groupe de 3. Chaque groupe reçoit plusieurs gouttes de collyre dans l'oeil droit pendant 5 jours selon le calendrier suivant :
Jour n° 1 : 5 gouttes administrées en 20 minutes.
Jour n° 2 : 5 gouttes administrées en 20 minutes, 2 fois par jour
Jour n° 3 : 5 gouttes administrées en 20 minutes, 4 fois par jour
Jour n° 4 : 5 gouttes administrées en 20 minutes, 6 fois par jour
Jour n° 5 : 5 gouttes administrées en 20 minutes, 8 fois par jour
Quantité de produit administré à chaque instillation : une goutte de 50 µL.

Les évaluations oculaires sont réalisées à l'aide d'un ophtalmoscope en utilisant l'échelle de Draize. Les deux yeux de chaque animal sont observés. Les lectures sont réalisées de la manière suivante :
Jour n° 1 : avant le traitement (état de base), puis 5 minutes, 30 minutes, 1 heure et 4 heures après la dernière instillation.
Jour 2 à 5 : juste avant la première instillation et 5 minutes après la dernière instillation du jour.

### Résultats:

| Yeux traités | | Conjonctive | | | | | |
|---|---|---|---|---|---|---|---|
| | | Rougeur | | Inflammation | | Décharge | |
| | | Jour 1 | Jours 2 à 5 | Jour 1 | Jours 2 à 5 | Jour 1 | Jours 2 à 5 |
| Formule A (latanoprost 0,005% sans conservateur) | Moyenne | 0,0 - 1,0 | 0,0 - 0,3 | 0,0 - 0,0 | 0,0 - 0,0 | 0,0 - 0,0 | 0,0 - 0,0 |
| | Score Max | 2 | 1 | 0 | 0 | 0 | 0 |
| | Nombre d'animaux avec effet | 2/3 | 2/3 | 0/3 | 0/3 | 0/3 | 0/3 |
| Formule B (travoprost 0,004% sans conservateur) | Moyenne | 0,3-1,0 | 0,0-1,0 | 0,0-0,0 | 0,0-0,0 | 0,0-0,3 | 0,0-0,0 |
| | Score Max | 2 | 2 | 0 | 0 | 0 | 0 |
| | Nombre d'animaux avec effet | 3/3 | 2/3 | 0/3 | 0/3 | 1/3 | 0/3 |
| Xalatan® (latanoprost 0,005% avec conservateur) | Moyenne | 0,3-1,7 | 0,0-0,7 | 0,0-0,0 | 0,0-0,0 | 0,0-0,0 | 0,0-0,0 |
| | Score Max | 2 | 1 | 0 | 0 | 0 | 0 |
| | Nombre d'animaux avec effet | 3/3 | 2/3 | 0/3 | 0/3 | 0/3 | 0/3 |
| NaCl 0,9 % (solution saline = blanc) | Moyenne | 0,0-0,0 | 0,0-0,3 | 0,0-0,0 | 0,0-0,0 | 0,0-0,0 | 0,0-0,0 |
| | Score Max | 0 | 1 | 0 | 0 | 0 | 0 |
| | Nombre d'animaux avec effet | 0/3 | 1/3 | 0/3 | 0/3 | 0/3 | 0/3 |

*Formule de Latanoprost sans conservateur:* Après plusieurs instillations répétées dans l'oeil droit, la somme de la rougeur conjonctivale est de 6/108 pour l'oeil droit pour 0 dans l'oeil gauche. Les sommes de l'inflammation conjonctivale et de la décharge conjonctivale sont de 0 pour chaque oeil. La somme des effets sur la cornée et l'iris est nulle, pour chaque oeil.

*Formule de Travoprost sans conservateur :* Après plusieurs instillations répétées dans l'oeil droit, la somme de la rougeur conjonctivale est de 11/108 pour l'oeil droit pour 0 dans l'oeil gauche. La somme de l'inflammation conjonctivale est de 0 pour chaque oeil. La somme de la décharge conjonctivale est de 1/108 pour l'oeil droit et de 0 pour l'oeil gauche. La somme des effets sur la cornée et l'iris est nulle, pour chaque oeil.

*Formule de Latanoprost avec conservateur (Référence* / *Xalatan®) :* Après plusieurs instillations répétées dans l'oeil droit, la somme de la rougeur conjonctivale est de 18/108 pour l'oeil droit pour 0 dans l'oeil gauche. Les sommes de l'inflammation conjonctivale et de la décharge conjonctivale sont de 0 pour chaque oeil. La somme des effets sur la cornée et l'iris est nulle, pour chaque oeil.

*Témoin négatif (Solution de NaCl à 0,9* %*)* : Après plusieurs instillations répétées dans l'oeil droit, la somme de la rougeur conjonctivale est de 1/108 pour l'oeil droit pour 0 dans l'oeil gauche. Les sommes de l'inflammation conjonctivale et de la décharge conjonctivale sont de 0 pour chaque oeil. La somme des effets sur la cornée et l'iris est nulle, pour chaque oeil.

### Conclusion :

En suivant ce protocole de plusieurs instillations répétées de différents collyres dans l'oeil droit de lapin albinos pendant 5 jours, les deux formules de prostaglandines sans conservateur induisent approximativement les mêmes réactions oculaires, qui sont légèrement plus faibles que celles obtenues pour la solution de référence (Xalatan®). Les résultats sont considérés comme valides du fait de la réponse obtenue avec le témoin négatif (solution saline). Il est ainsi conclu que dans les conditions de l'étude, les deux collyres de prostaglandines sans conservateur présentent une très bonne tolérance oculaire.

### Exemple 10 : Etude comparative de la pharmacocinétique oculaire chez le lapin pigmenté

Objectif : Comparer la rémanence du latanoprost (forme acide) dans deux tissus oculaires, après instillation d'une dose unique de deux collyres dosés à 0,005% (Latanoprost isopropyl ester). Le collyre Xalatan®, est considéré ici comme le produit de référence. L'étude a ainsi pour but d'évaluer si la formule A présente un profil de cinétique oculaire équivalent à celui du produit de référence.

### Méthodologie :

Etude réalisée sur deux groupes de 18 lapins pigmentés (Fauves de Bourgognes), après instillation unique d'une goutte d'une formulation topique ophtalmique de Latanoprost à 0,005% dans l'oeil droit. L'oeil gauche est utilisé comme témoin négatif. Le premier groupe reçoit le collyre sans conservateur (Formule A), tandis que le deuxième groupe reçoit le produit de référence avec conservateur (Xalatan®). Taille de la goutte instillée : 50 µL. Un dosage du Latanoprost (forme acide) par CLHP-MS est réalisé après extraction des deux tissus oculaires : la conjonctive et la cornée. Le dosage est réalisé à différents temps. 6 lapins par groupe sont testés à chaque temps d'analyse.

### Résultats :

| Temps (heures) | Formule A (sans conservateur) | | Xalatan® (référence, avec conservateur) | |
|---|---|---|---|---|
| | Cornée | Conjonctive | Cornée | Conjonctive |
| 0,5 | 100 % | 100 % | 100 % | 100 % |
| 6 | 5,3%+/-5% | 1,0%+/-5% | 5,5% +/- 5 % | 1,2% +/- 5% |
| 24 | 0,2 % +/- 5 % | 0,3 % +/- 5% | 0,1% +/- 5% | 0,2% +/- 5 % |

*Conclusion :* Les cinétiques de concentration oculaire du latanoprost après instillation unique d'une goutte d'une formule topique ophtalmique à 0,005% sont équivalentes pour les deux produits, dans les conditions de l'étude.

## Revendications

1. Solution ophtalmique dépourvue d'agent conservateur anti-microbien comprenant en tant que principe actif au moins une prostaglandine, ainsi qu'en tant qu'agent solubilisant un tensio-actif, **caractérisé en ce que** l'agent solubilisant est le polyoxyl-15-hydroxystearate.

2. Solution selon la revendication 1, **caractérisée en ce que** la concentration de l'agent solubilisant dans la solution est comprise entre 1 et 20 g/l, avantageusement entre 2 et 10g/l.

3. Solution selon la revendication 1, **caractérisée en ce que** la concentration en prostaglandine dans la solution est comprise entre 0.02 et 1.5 g/l.

4. Solution selon la revendication 1, **caractérisée en ce que** la prostaglandine est choisie dans le groupe comprenant 17-phenyl-13,14 dihydro trinor Prostaglandin F_{2α} isopropyl ester (latanoprost), 20-ethyl Prostaglandin F_{2α}, (+)- Fluprostenol isopropyl ester (travoprost), 17-phenyl trinor Prostaglandin F_{2α} amide, 17-phenyl-13,14 dihydro trinor Prostaglandin F_{2α} ethyl amide (bimatoprost), tafluprost Prostaglandin F_{2α} ethanolamide, bimatoprost (freeacid)-d₄, bimatoprost-d₄, latanoprost ethyl amide, 13,14 dihydro-15-keto-20-ethyl Prostaglandin F_{2α} (unoprostone), 13,14 dihydro-15-keto-20-ethyl Prostaglandin F_{2α} isopropyl ester (unoprostone isopropyl ester).

5. Solution selon la revendication 1, **caractérisée en ce qu'**elle contient en outre un agent anti-glaucomateux choisi dans le groupe comprenant les béta-bloquants, les inhibiteurs d'anhydrase carbonique et les agonistes alpha-adrénergiques.

6. Solution selon la revendication 5, **caractérisée en ce que** l'agent anti-glaucomateux représente entre 0,1 et 0,5% en poids de la solution.

7. Solution selon la revendication 1, **caractérisée en ce qu'**elle contient en outre un additif choisi dans le groupe comprenant les agents isotonisants, les agents antioxydants et les systèmes tampons.

8. Flacon à usage unique ou multidose réalisé en PEBD sans additifs comprenant la solution ophtalmique objet de l'une des revendications 1 à 7.

9. Utilisation de la solution selon l'une des revendications 1 à 7 pour la fabrication d'un médicament destiné au traitement du glaucome, capable de réduire la pression intraoculaire et/ou capable d'assurer la neuroprotection des tissus rétiniens.

10. Utilisation de la solution selon la revendication 9 consistant à administrer à l'homme ou à l'animal, par voie topique, une goutte par jour de ladite solution dans chaque oeil.

## Claims

1. Ophthalmic solution without antimicrobial preservative including as active substance at least one prostaglandin and as solubilizing agent, a surfactant **characterized in that** the solubilizer is polyoxyl-15-hydroxystearate.

2. Solution according to claim 1, **characterized in that** the concentration of the solubilizer in the solution is between 1 and 20 g/l, to advantage between 2 and 10 g/l.

3. Solution according to claim 1, **characterized in that** the prostaglandin concentration in the solution is between 0.02 and 1.5 g/l.

4. Solution according to claim 1 **characterized in that** the prostaglandin is selected from the group comprising 17-phenyl-13,14 dihydro trinor prostaglandin F_{2α} isopropyl ester (latanoprost), 20-ethyl prostaglandin F_{2α}, (+)- fluprostenol isopropyl ester (travoprost), 17-phenyl trinor prostaglandin F_{2α} amide, 17-phenyl-13,14 dihydro trinor prostaglandin F_{2α} ethyl amide (bimatoprost), tafluprost prostaglandin F_{2α} ethanolamide, bimatoprost (free acid)-d₄, bimatoprost-d₄, latanoprost ethyl amide, 13,14 dihydro-15-keto-20-ethyl prostaglandin F_{2α} (unoprostone), 13,14 dihydro-15-keto-20-ethyl prostaglandin F_{2α} isopropyl ester (unoprostone isopropyl ester).

5. Solution according to claim 1, **characterized in that** it contains in addition an antiglaucoma agent selected from the group including the beta-blockers, carbonic anhydrase inhibitors and alpha-adrenergic agonists.

6. Solution according to claim 5, **characterized in that** the antiglaucoma agent forms between 0.1 and 0.5% by weight of the solution.

7. Solution according to claim 1, **characterized in that** it contains in addition an additive selected from the group including isotonicity agents, antioxidants and buffer systems.

8. Single-use or multi-dose vial produced in additive-free LDPE containing the ophthalmic solution forming the subject of one of the claims 1 to 7.

9. Use of the solution according to one of the claims 1 to 7 for the manufacture of a medicinal product intended to treat glaucoma, capable of reducing intraocular pressure and/or capable of providing neuroprotection to retinal tissues.

10. Use of the solution according to claim 9 consisting of administering to humans or animals, via the topical route, one drop per day of the said solution into each eye.

## Patentansprüche

1. Von antimikrobiellem Konservierungsmittel freie ophtalmische Lösung, die als Wirkstoff mindestens ein Prostaglandin sowie als Solubilisierungsmittel ein Tensid umfasst, **dadurch gekennzeichnet, dass** das Solubilisierungsmittel Polyoxyl-15-hydroxystearat ist.

2. Lösung nach Anspruch 1, **dadurch gekennzeichnet, dass** die Konzentration des Solubilisierungsmittels in der Lösung zwischen 1 und 20 g/l, vorteilhafter Weise zwischen 2 und 10 g/l beträgt.

3. Lösung nach Anspruch 1, **dadurch gekennzeichnet, dass** die Konzentration des Prostaglandins in der Lösung zwichen 0,02 und 1,5 g/l beträgt.

4. Lösung nach Anspruch 1, **dadurch gekennzeichnet, dass** das Prostaglandin ausgewählt ist aus der Gruppe, die umfasst: 17-Phenyl-13,14-dihydro-trinor-Prostaglandin-F_{2α}-isopropylester (Latanoprost), 20-Ethyl-Prostaglandin-F_{2□}, (+)-Fluprostenol-isopropylester (Travoprost),17-Phenyl-trinor-Prostaglandin-F_{2□}-amid, 17-Phenyl-13,14-dihydro-trinor-Prostaglandin-F_{2□}-ethylamid (Bimatoprost), Tafluprost-Prostaglandin-F_{2□}-ethanolamid, Bimatoprost-(freie Säure)-d₄, Bimatoprost-d₄, Latanoprost-ethylamid, 13,14-Dihydro-15-keto-20-ethyl-Prostaglandin-F_{2□} (Unoproston), 13,14-Dihydro-15-keto-20-ethyl-Prostaglandin-F_{2□})-isopropylester (Unoproston-Isopropylester).

5. Lösung nach Anspruch 1, **dadurch gekennzeichnet, dass** sie außerdem ein Antiglaukom-Mittel enthält, das ausgewählt ist aus der Gruppe, die die Betablocker, die Carboanhydrasehemmer und die alpha-adrenergen Agonisten umfasst.

6. Lösung nach Anspruch 5, **dadurch gekennzeichnet, dass** das Antiglaukom-Mittel zwischen 0,1 und 0,5 Gew.-% der Lösung darstellt.

7. Lösung nach Anspruch 1, **dadurch gekennzeichnet, dass** sie außerdem einen Zusatz enthält, der ausgewählt ist aus der Gruppe, die die Isotonisierungsmittel, die antioxidierenden Mittel und die Puffersysteme umfasst.

8. Einmalgebrauch- oder Mehrdosen-Flasche aus PEBD ohne Zusätze, umfassend die ophtalmische Lösung, die Gegenstand eines der Ansprüche 1 bis 7 ist.

9. Verwendung der Lösung nach einem der Ansprüche 1 bis 7 für die Herstellung eines Medikaments, das für die Behandlung des Glaukoms bestimmt ist, den Augeninnendruck reduzieren kann und/oder den Neuroschutz der Netzhautgewebe gewährleisten kann.

10. Verwendung der Lösung nach Anspruch 9, die darin besteht, dass dem Mensch oder dem Tier auf topischem Weg ein Tropfen der Lösung täglich in jedes Auge verabreicht wird.
